(19) Europäisches Patentamt
European Patent Office
Office européen des brevets

(11) Numéro de publication: **0 094 303**
**B1**

# (12) FASCICULE DE BREVET EUROPEEN

(45) Date de publication du fascicule du brevet: **06.11.85**

(21) Numéro de dépôt: **83400905.2**

(22) Date de dépôt: **05.05.83**

(51) Int. Cl.⁴: **C 07 D 417/12**, C 07 D 277/48, A 61 K 31/425, A 61 K 31/44

(54) **Amides, leurs sels, procédé pour leur préparation et compositions pharmaceutiques les renfermant.**

(30) Priorité: **10.05.82 FR 8208097**
**13.08.82 FR 8214126**

(43) Date de publication de la demande:
**16.11.83 Bulletin 83/46**

(45) Mention de la délivrance du brevet:
**06.11.85 Bulletin 85/45**

(84) Etats contractants désignés:
**AT BE CH DE FR GB IT LI LU NL SE**

(56) Documents cités:
**EP-A-0 010 893**
**EP-A-0 045 155**
**FR-A-2 103 543**
**FR-A-2 220 271**
**FR-A-2 220 273**
**GB-A-2 076 397**
**US-A-4 165 378**

(73) Titulaire: **SANOFI, société anonyme, 40, Avenue George V, F-75008 Paris (FR)**

(72) Inventeur: **Nisato, Dino, Viale Golgi 80, Pavia (IT)**
Inventeur: **Boveri, Sergio, Via Cesare Abba 5, Monza-Milano (IT)**
Inventeur: **Roncucci, Romeo, 20 rue Tournefort, F-75005 Paris (FR)**
Inventeur: **Carminati, Paolo, Via Pacini 24, Milano (IT)**

(74) Mandataire: **Gillard, Marie-Louise et al, Cabinet Beau de Loménie 55, Rue d'Amsterdam, F-75008 Paris (FR)**

**EP 0 094 303 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des amides ayant une activité bloquant les récepteurs $H_2$ de l'histamine, leurs sels, un procédé pour leur préparation et des compositions pharmaceutiques les renfermant en tant qu'ingrédients actifs.

Après la subdivision des récepteurs de l'histamine en récepteurs $H_1$ (Ash et Schild, Brit. J. Pharmac. Chemother. 1966, 27, 427) et récepteurs $H_2$ (Black et al., Nature 1972, 236, 385) et la découverte que le blocage sélectif des récepteurs $H_2$ provoque une inhibition de la sécrétion gastrique, de nombreux produits ont été proposés comme antagonistes des récepteurs $H_2$ de l'histamine, ci-après indiqués $H_2$-bloquants. Ainsi, les composés ayant reçu les Dénominations Communes Internationales burimamide, métiamide, cimétidine, ranitidine, tiotidine, étintidine, oxmétidine ont fait l'objet d'un grand nombre de publications scientifiques.

Tous les produits ci-dessus sont caractérisés par la présence dans leur molécule de la structure suivante:

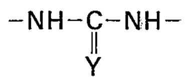

où Y représente un atome d'oxygène ou de soufre, ou bien un groupe $N-CN$ ou $CH-NO_2$, ladite structure étant linéaire ou incluse dans un cycle comme dans le cas de l'oxmétidine. Les produits ci-dessus sont donc tous caractérisés par la présence de deux atomes d'azote gémineux par rapport à un atome de carbone.

La cimétidine, 2-cyano-1-méthyl-3-[2-[[(5-méthyl-imidazol-4-yl)méthyl]thio]éthyl]guanidine ayant la structure I où Y est $N-CN$, et la ranitidine, N[2-[[5-[(diméthylamino)méthyl]furfuryl]-thio]N'-méthyl-2-nitro-1,1-éthènediamine ayant la structure I où Y est $CH-NO_2$, sont déjà utilisées en thérapeutique pour le traitement de l'ulcère gastrique et duodénal.

Le brevet belge N° 888602 décrit et revendique des amides ayant la formule

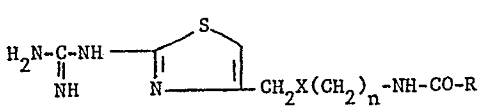 (II)

dans laquelle n représente un nombre entier de 2 à 4, R représente hydrogène, un groupe alkyle en $C_1$-$C_6$ (portant éventuellement comme substituant un radical hydroxyle, cyano, amino ou phénoxy), un radical cycloalkyle en $C_3$-$C_6$, un radical alcényle en $C_2$-$C_6$ (portant éventuellement comme substituant un radical phényle ou alcoxycarbonyle en $C_2$-$C_7$), un radical alkadiényle en $C_4$-$C_7$, un radical aryle en $C_6$-$C_{12}$ (portant éventuellement 1 à 3 substituants choisis entre les atomes d'halogène et radicaux cyano, nitro, amino, dialkylamino en $C_2$-$C_{10}$, tétrazolyle, hydroxyle, alkoxy en $C_1$-$C_6$, benzoyloxy, alkoxycarbonyle en $C_2$-$C_7$, sulfamoyle, alkylsulfonyle en $C_1$-$C_6$, alkanesulfonamido en $C_1$-$C_6$ et alkanoyle en $C_1$-$C_6$) ou un hétérocycle pentagonal ou hexagonal (portant éventuellement comme substituant un atome d'halogène ou un radical alkyle en $C_1$-$C_6$ ou phényle), et X représente une liaison simple ou un radical thia de même que leurs sels d'addition d'acides pharmaceutiquement acceptables, qui sont d'utiles agents contre l'ulcère peptique ou d'utiles antagonistes des récepteurs $H_2$ de l'histamine.

Parmi les produits décrits dans le brevet ci-dessus, le composé de formule II où R=hydrogène, n=2 et X=thio, à savoir le 2-guanidino-4-[(2-formamido)éthylthiométhyl]thiazole, sous forme de maléate, montre une valeur de DE50 de 0,25 mg/kg i.v. dans l'activité d'inhibition de la sécrétion d'acide de l'estomac chez le rat (Ghosh et al., Brit. J. Pharmacol. 1958, 13, 54).

Le brevet belge ci-dessus, plus particulièrement, comprend les composés de formule II ci-dessus où R est un groupe pyridyle ou un groupe phényle substitué par un groupe alkanesulfonamido en $C_1$-$C_6$ et décrit spécifiquement le composé où R est 3-pyridyle ou un groupe $p-CH_3-SO_2-NH-C_6H_4$.

On a maintenant trouvé qu'un nicotinamide 1-oxyde ne présentant pas la structure I ci-dessus possède une très bonne action antagonisant les récepteurs $H_2$ de l'histamine.

On a aussi trouvé que de nouveaux amidobenzamides possèdent une bonne action antagonisant les récepteurs $H_2$ de l'histamine, supérieure à celle des composés analogues décrits dans le brevet belge N° 888602.

On a également trouvé, d'une façon surprenante, que l'activité $H_2$-bloquante ne se produit à un niveau satisfaisant que lorsque le groupe amido est dans la position méta du noyau phényle des benzamides.

Ainsi, la présente invention, selon un de ses aspects, a pour objet des amides de formule

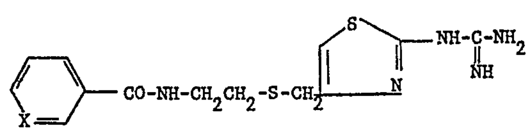 (III)

dans laquelle X représente un groupe $\geqslant N \rightarrow O$ ou un groupe $\geqslant C-NH-A-B$, où A représente un groupe CO ou $SO_2$ et B représente un groupe alkyle contenant de 1 à 6 atomes de carbone, un groupe phényle, un groupe pyridyle, un groupe pyridyle 1-oxyde, un groupe pyrazinyle ou un groupe thiényle, ainsi que leurs sels pharmaceutiquement acceptables.

Les sels pharmaceutiquement acceptables comprennent les sels non toxiques dérivés d'acides minéraux ou organiques salifiant une ou les deux fonctions basiques présentes dans la molécule des composés de formule III, tels que le chlorhydrate, le bromhydrate, le sulfate, le succinate, le tartrate, le citrate, le fumarate, le maléate, le 4,4'-méthylènebis-(3-hydroxy-2-naphtoate), ci-après désigné pamoate, le 2-naphtalènesulfonate, ci-après désigné napsylate, le méthanesulfonate, ci-après désigné mésylate, le p-toluènesulfonate, ci-après désigné tosylate, et similaires.

Selon un autre de ses aspects, la présente invention a pour objet un procédé pour la préparation des composés de formule III ci-dessus, ledit procédé étant caractérisé en ce que l'on traite la 2-(2-guanidinothiazol-4-ylméthylthio)éthylamine de formule

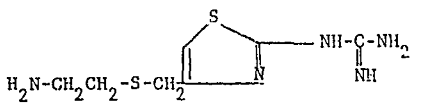

(IV)

avec un dérivé fonctionnel d'un acide de formule

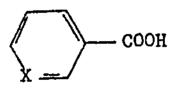

(V)

dans laquelle X est tel que défini ci-dessus, dans un solvant organique à une température comprise entre 0°C et la température d'ébullition du solvant employé et l'on transforme éventuellement le produit ainsi obtenu dans ses sels pharmaceutiquement acceptables.

Comme dérivé fonctionnel approprié, on peut utiliser l'acide libre activé, l'anhydride, une anhydride mixte, le chlorure ou un ester actif.

Un dérivé fonctionnel préféré de l'acide de formule V ci-dessus est représenté par la formule suivante

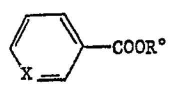

(VI)

dans laquelle X est tel que défini ci-dessus et R° représente un groupe nitrophényle, méthoxyphényle, trityle, benzhydryle.

Les composés de formule VI ci-dessus sont connus en littérature où ils peuvent être aisément préparés par réaction de l'acide V avec l'alcool ou le phénol approprié en présence d'un agent de condensation tel que la dicyclohexylcarbodiimide dans un solvant tel que le chlorure de méthylène.

La température de réaction peut varier entre 0°C et le point d'ébullition du solvant employé, mais en général on opère à la température ambiante ou à 30-50°C. Il peut être préférable de conduire la réaction au froid lorsqu'elle est exothermique, comme dans le cas où on utilise le chlorure en tant que dérivé fonctionnel de l'acide de formule V.

Comme solvant de réaction, on utilise de préférence un alcool, tel que le méthanol, ou un solvant halogéné tel que le chlorure de méthylène, le dichloroéthane, le chloroforme et similaires, mais d'autres solvants organiques compatibles avec les réactifs employés, par exemple l'acétonitrile, le dioxane, le tétrahydrofuranne ou un hydrocarbure tel que l'hexane peuvent être également employés.

La réaction peut être conduite en présence d'un accepteur de protons, par exemple d'un carbonate alcalin ou d'une amine tertiaire, dans le cas où de l'acide chlorhydrique, ou un autre acide, se libère pendant la réaction, mais cet accepteur de protons n'est pas indispensable pour l'obtention du produit final.

La réaction est assez rapide; en général, après 2-4 h à la température ambiante ou à 30-50°C la réaction est achevée et l'amide de formule III obtenu est isolé selon les techniques conventionnelles sous forme de base libre ou d'un de ses sels.

La base libre peut être transformée dans un de ses sels pharmaceutiquement acceptables par traitement avec une solution de l'acide convenable dans un solvant organique. Si l'amide III est isolé sous forme de sel, la base libre correspondante peut être libérée à l'aide d'un hydroxyde ou d'un carbonate alcalin.

Les nouveaux composés de formule III de la présente invention, ainsi que leurs sels pharmaceutiquement acceptables, agissent comme antagonistes sélectifs des récepteurs $H_2$ de l'histamine en inhibant sélectivement la sécrétion gastrique et sont donc utiles pour le traitement de la maladie ulcéreuse.

La sélectivité de l'activité des produits de la présente invention vers les récepteurs du type $H_2$ est confirmée par l'absence d'activité du type $H_1$ dans le test de la contraction provoquée par l'histamine sur l'iléon isolé de cobaye.

L'activité antagoniste des amides de la présente invention vers les récepteurs $H_2$ de l'histamine a été confirmée dans le test de l'activité antisécrétoire basé sur l'antagonisme pour l'hypersécrétion provoquée par l'histamine chez le rat atropinisé, selon la méthode de Ghosh et Schild (Brit. J. Pharmacol. 1958, 13, 54), modifiée selon Black et al. (Nature 1972, 236, 385). Selon ce test, on provoque une hypersécrétion acide gastrique par infusion intraveineuse d'une dose submaximale d'histamine équivalant à 15 mcmol/kg/h et l'on mesure la sécrétion gastrique par perfusion d'une solution physiologique à une vitesse constante dans l'estomac de l'animal.

Le tableau I montre pour trois composés représentatifs de la présente invention décrits, respectivement, aux exemples 3, 4 et 1 ci-dessous et ici désignés par leur numéro de code SR 57938 A, SR 57963 A et SR 57957 A, et pour quatre produits de référence, la 2-cyano-1-méthyl-3-[2-[[(5-méthylimidazol-4-yl)méthyl]thio]éthyl]guanidine, ci-après désignée par sa Dénomination Commune Internationale cimétidine, la N-[2-[[5-[(diméthylamino)méthyl]furfuryl]thio]éthyl]-N'-méthyl-2-nitro-1,1-éthènediamine, ci-après désignée par sa Dénomination Commune Internationale ranitidine et les 4-méthanesulfonamido-N-[2-(2-guanidinothiazol-4-ylméthylthio)éthyl]benzamide et N-[2-(2-guanidinothiazol-4-ylméthylthio)-éthyl]nicotinamide décrits dans le brevet belge N° 888602 cité ci-dessus, ci-après désignés, respectivement, Composé A et Composé B, la dose (en mcmol/kg par voie veineuse en dose unique) qui inhibe de 50% l'hypersécrétion gastrique provoquée par l'histamine (DI50), qui représente l'index de l'activité $H_2$-bloquante gastrique.

*(Tableau en page suivante)*

De ce tableau il ressort que les composés représentatifs de la présente invention sont tous plus actifs que les composés de référence; plus particu-

*Tableau I*

| Composé | DI50 (mcmol/kg) | Puissance relative (cimétidine=1) |
|---|---|---|
| Cimétidine | 0,95 | 1,00 |
| Ranitidine | 0,25 | 3,80 |
| Composé A | 1,66 | 0,57 |
| Composé B | 0,28 | 3,39 |
| SR 57 938 A | 0,15 | 6,33 |
| SR 57 963 A | 0,065 | 14,6 |
| SR 57 957 A | 0,10 | 9,5 |

lièrement, les composés représentatifs sont beaucoup plus actifs que le composé A. Cette découverte est très surprenante si l'on considère que la différence chimique entre le SR 57938 A et le Composé A n'est qu'une isomérie de position et que le SR 57938 A est 10 fois plus actif que le Composé A.

Par rapport à leur degré d'activité, les composés de la présente invention sont peu toxiques. Par exemple à une dose de 375 mg/kg par voie intrapéritonéale chez la souris, le SR 57957 A ne provoque pas de mortalité; sa DL50 est supérieure à 900 mcmol/kg, alors que, dans les mêmes conditions, le Composé B montre une DL50 de 342 mcmol/kg.

La présente invention, selon un autre de ses aspects, se réfère ainsi à des compositions pharmaceutiques renfermant, en tant qu'ingrédients actifs, les amides de formule III ci-dessus ou leurs sels d'addition acceptables du point de vue pharmaceutique.

Les compositions pharmaceutiques à action $H_2$-bloquante de la présente invention peuvent être formulées pour l'administration orale, sublinguale, sous-cutanée, intramusculaire, intraveineuse, transdermique ou rectale, en mélangeant les ingrédients actifs avec des supports pharmaceutiques classiques. Elles peuvent être administrées aux animaux et êtres humains dans le traitement de l'hypersécrétion gastrique et de l'ulcère peptique sous des formes unitaires d'administration.

Afin d'obtenir l'effet $H_2$-bloquant désiré, la dose de principe actif peut varier entre 1 et 100 mg/kg de poids du corps/d, de préférence 10 à 50 mg/kg de poids/d.

Chaque dose unitaire peut contenir de 10 à 1000 mg, de 100 à 500 de préférence, d'ingrédient actif en combinaison avec un support pharmaceutique. Cette dose unitaire peut être administrée 1 à 4 fois/d.

Les formes unitaires d'administration comprennent les comprimés, les gélules, les poudres, les granules et les solutions ou suspensions orales et les tablettes pour l'administration sublinguale, les suppositoires ainsi que les ampoules utiles pour une administration parentérale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues.

On peut enrober les comprimés de saccharose ou d'autres matières appropriées ou encore on peut les traiter de telle sorte qu'ils aient une activité prolongée ou retardée et qu'ils libèrent continuellement une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, tels que la polyvinylpyrrolidone et similaires, de même qu'avec des édulcorants ou des correcteurs du goût.

Pour une application rectale, on recourt à des suppositoires qui sont préparés avec des liants fondant à la température rectale, par exemple du beurre de cacao ou des polyéthylèneglycols.

Pour une administration orale en gouttes ou pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les exemples suivants illustrent l'invention sans toutefois la limiter.

*Exemple 1:*

On ajoute à portions, sous agitation et à la température de −30°C, 5 g de chlorhydrate du chlorure de nicotinoyle 1-oxyde à une solution de 6,1 g de chlorhydrate de 2-(2-guanidinothiazol-4-ylméthylthio)éthylamine et 30 ml de triéthylamine dans 100 ml de méthanol. On agite le mélange 15 min à −30°C et, ensuite, 1 h à la température ambiante, puis on filtre et on évapore le solvant sous pression réduite. On reprend le résidu avec 20 ml d'eau et la solution obtenue est traitée avec une solution concentrée d'hydroxyde de sodium. On extrait avec de l'acétate d'éthyle contenant 10% d'éthanol, on évapore le solvant et l'on purifie l'huile brute par chromatographie sur $SiO_2$ en utilisant comme éluant un mélange méthanol: chloroforme 1:4. On obtient ainsi 3,8 g de N-[-2-(2-guanidinothiazol-4-ylméthylthio)éthyl]-3-pyridinecarboxamide 1-oxyde (SR 57957) sous forme d'une huile jaune pâle.

H RMN · Solvant: DMSO-$d_6$ · $S_{TMS}$ (ppm): 2,68 (2H, m→t); 3,44 (2H, m→g); 3,63 (2H, s); 6,48 (1H, s); 6,83 (sb); 7,46 (1H, dd, $J_1$=6Hz, $J_2$= 8 Hz); 7,68 (1H, ddd, $J_1$=8 Hz), $J_2 \simeq J_3 \simeq$ 2Hz); 8,30 (1H, ddd, $J_1$=6Hz, $J_2 \simeq J_3 \simeq$ 2Hz); 8,53 (1H,

dd, $J_1 \simeq J_2 \simeq 2Hz$); 8,83 (1H, tb). Les signaux à 8,83 ppm et 6,83 ppm disparaissent après l'échange avec $D_2O$.

On dissout le produit ainsi obtenu dans 25 ml d'éthanol 95% et on ajoute à la solution ainsi obtenue 30 ml d'acide fumarique dissous dans 30 ml d'éthanol à 95%. Par cristallisation du précipité de 30 ml d'eau, on obtient 2,6 g de fumarate neutre de N-[2-(2-guanidinothiazol-4-ylméthylthio)éthyl]-3-pyridinecarboxamide 1-oxyde (SR 57957 A); F. 220-222°C (décomposition).

*Exemple 2:*

A une solution de 2,2 g de fumarate neutre de N-[2-(2-guanidinothiazol-4-ylméthylthio)éthyl]-3-pyridinecarboxamide 1-oxyde dans 15 ml d'eau on ajoute de l'hydroxyde de sodium jusqu'à réaction nettement basique. On extrait avec de l'acétate d'éthyle contenant 10% d'éthanol, on sèche la phase organique sur du sulfate de sodium anhydre et on l'évapore à sec. On obtient ainsi 1,7 g d'un liquide visqueux qui cristallise lentement et solidifie en devenant un solide amorphe. La structure du N-[2-(2-guanidinothiazol-4-ylméthylthio)éthyl]-3-pyridinecarboxamide 1-oxyde ainsi obtenu a été confirmée par RMN et IR.

*Exemple 3:*

On chauffe à 40°C sous agitation pendant 4 h une solution de 0,015 mol de 2-(2-guanidinothiazol-4-ylméthylthio)éthylamine, de 0,015 mol de 3-méthanesulfonamidobenzoate de 4-nitrophényle (F. 162-164°C, préparé de l'acide 3-méthanesulfonamidobenzoïque et du 4-nitrophénol dans du chlorure de méthylène en présence de dicyclohexylcarbodiimide) et de 0,035 mol de triéthylamine dans 150 ml de méthanol, puis on évapore le solvant sous pression réduite et on reprend le résidu dans 20 ml d'eau et 100 ml d'acétate d'éthyle. On sèche la phase organique sur du sulfate de sodium anhydre, on évapore le solvant sous pression réduite et on dissout le résidu dans 50 ml d'acétate d'éthyle chaud. On filtre et on acidifie le filtrat avec du gaz chlorhydrique en isopropanol. On obtient ainsi le chlorhydrate de 3-méthanesulfonamido-N-[2-(2-guanidinothiazol-4-ylméthylthio)éthyl]benzamide qui, après recristallisation de 20 ml d'eau, donne 2 g du produit monohydraté, SR 57938 A; F. 117-120°C.

*Exemple 4:*

On agite à la température de 40°C pendant 90 min un mélange de 5,5 g de dichlorhydrate de 2-(2-guanidinothiazol-4-ylméthylthio)éthylamine, 3,65 g de triéthylamine et 3,7 g de 3-benzènesulfonamidobenzoate de 4-nitrophényle (F. 175-178°C, préparé de l'acide 3-benzènesulfonamidobenzoïque et du 4-nitrophénol dans du chlorure de méthylène en présence de dicyclohexylcarbodiimide), puis on l'évapore à sec sous pression réduite. On reprend le résidu dans 150 ml d'acétate d'éthyle et on le lave deux fois avec 10 ml d'eau. On sèche la solution sur du sulfate de sodium anhydre, on l'évapore et le résidu est chromatographié sur silice avec un mélange chloroforme: éthanol 4:1. On obtient 3,8 g de 3-benzène-sulfonamido-N-[2-(2-guanidinothiazol-4-ylméthylthio)éthyl]benzamide sous forme d'une huile qui est dissoute en éthanol absolu et traitée avec de l'acide fumarique en éthanol absolu. On obtient 3,6 g de produit brut qui, après cristallisation de 40 ml d'éthanol à 95%, donne 2,4 g de fumarate neutre de 3-benzènesulfonamido-N-[2-(2-guanidinothiazol-4-ylméthylthio)éthyl]benzamide, SR 57963 A, $C_{20}H_{22}N_6O_3S_4 \cdot ½\ C_4H_4O_4$; F. 139-141°C.

De la même façon, par réaction du dichlorhydrate de 2-(2-guanidinothiazol-4-ylméthylthio)éthylamine avec, respectivement, le 3-acétamidobenzoate et le 3-nicotinoylaminobenzoate de 4-nitrophényle, on obtient:

– le fumarate neutre de 3-acétamido-N-[2-(2-guanidinothiazol-4-yl-méthylthio)éthyl]benzamide

– le fumarate neutre de 3-nicotinoylamino-N-[2-(2-guanidinothiazol-4-ylméthylthio)éthyl]-benzamide.

*Exemple 5:*

Comprimés à base d'un des composés décrits aux exemples 1 à 4, ayant la composition suivante:

| | |
|---|---|
| principe actif | 100 mg |
| lactose | 70 mg |
| fécule de pommes de terre | 40 mg |
| polyvinylpyrrolidone | 8 mg |
| stéarate de magnésium | 2 mg |

On humecte le mélange de la substance active avec le lactose et la fécule de pommes de terre d'une solution alcoolique de polyvinylpyrrolidone à 15%, on fait passer le granulé formé à travers un tamis de 1 mm, on le mélange avec le stéarate de magnésium et on forme des comprimés par compression. Poids d'un comprimé: 220 mg.

*Exemple 6:*

On recouvre de façon connue les comprimés fabriqués comme décrit à l'exemple 5 d'un enrobage pour dragées consistant essentiellement en sucre et talc et on polit les dragées finies par de la cire d'abeilles. Poids d'une dragée: 300 mg.

*Exemple 7:*

Gélules à base d'un des composés décrits aux exemples 1 à 4, ayant la composition suivante:

| | |
|---|---|
| principe actif | 200 mg |
| amidon de maïs | 90 mg |
| talc | 10 mg |

On mélange intimement le principe actif et les excipients et l'on introduit le mélange ainsi obtenu dans des gélules de gélatine de dimension 1. Contenu d'une gélule: 300 mg.

*Exemple 8:*

Suppositoires à base d'un des composés décrits aux exemples 1 à 4, ayant la composition suivante:

| | |
|---|---|
| principe actif | 300 mg |
| masse pour suppositoires (Witepsol W 45) | 1450 mg |

On met en suspension la substance active finement pulvérisée dans la masse pour suppositoires

à 37°C et on verse le mélange dans des moules légèrement refroidis au préalable. Poids d'un suppositoire: 1750 mg.

*Exemple 9:*

Comprimés à base d'un des composés décrits aux exemples 1 à 4, ayant la composition suivante:

| | |
|---|---|
| principe actif | 150 mg |
| cellulose microcristalline | 75 mg |
| lactose | 100 mg |
| stéarate de magnésium | 7 mg |
| talc | 18 mg |

On fait passer les poudres à travers un tamis de 0,3 mm, puis on mélange les ingrédients jusqu'à ce qu'on obtienne un mélange homogène qui est comprimé et granulé. Les granules ainsi obtenus sont utilisés pour former des comprimés par compression. Poids d'un comprimé: 350 mg.

*Exemple 10:*

En opérant comme décrit à l'exemple 9, on prépare des comprimés à base d'un des composés décrits aux exemples 1 à 4, ayant la composition suivante:

| | |
|---|---|
| principe actif | 350 mg |
| cellulose microcristalline | 100 mg |
| lactose | 125 mg |
| stéarate de magnésium | 10 mg |
| talc | 15 mg |

Poids d'un comprimé: 600 mg.

*Exemple 11:*

En opérant comme décrit à l'exemple 7, on prépare des gélules contenant, respectivement, 100 et 150 mg de SR 57957 A.

**Revendications** pour les Etats contractants BE, CH, DE, FR, GB, IT, LI, LU, NL, SE

1. Amides de formule

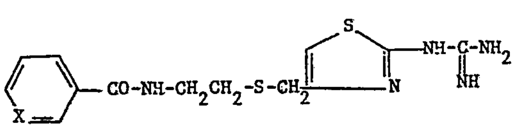

dans laquelle X représente un groupe ⩾N→O ou un groupe ⩾C−NH−A−B, où A représente un groupe CO ou $SO_2$ et B représente un groupe alkyle contenant de 1 à 6 atomes de carbone, un groupe phényle, un groupe pyridyle, un groupe pyridyle 1-oxyde, un groupe pyrazinyle ou un groupe thiényle, ainsi que leurs sels pharmaceutiquement acceptables.

2. N-[2-(2-guanidinothiazol-4-ylméthylthio)-éthyl]-3-pyridinecarboxamide 1-oxyde ainsi que ses sels pharmaceutiquement acceptables.

3. Fumarate neutre de N-[2-(2-guanidinothiazol-4-ylméthylthio)éthyl]-3-pyridinecarboxamide-1-oxyde.

4. 3-Méthanesulfonamido-N-[2-(2-guanidinothiazol-4-ylméthylthio)éthyl]benzamide et ses sels pharmaceutiquement acceptables.

5. 3-Benzènesulfonamido-N-[2-(2-guanidinothiazol-4-ylméthylthio)éthyl]benzamide et ses sels pharmaceutiquement acceptables.

6. Procédé pour la préparation d'amides de formule

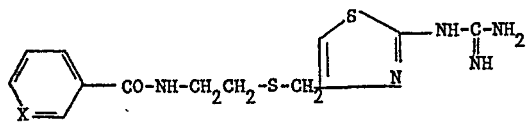

dans laquelle X représente un groupe ⩾N→O ou un groupe ⩾C−NH−A−B, où A représente un groupe CO ou $SO_2$ et B représente un groupe alkyle contenant de 1 à 6 atomes de carbone, un groupe phényle, un groupe pyridyle, un groupe pyridyle 1-oxyde, un groupe pyrazinyle ou un groupe thiényle, ainsi que de leurs sels pharmaceutiquement acceptables, caractérisé en ce que l'on traite la 2-(2-guanidinothiazol-4-ylméthylthio)éthylamine de formule

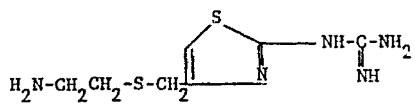

avec un dérivé fonctionnel d'un acide de formule

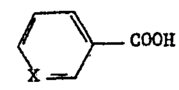

dans laquelle X est tel que défini ci-dessus, dans un solvant organique à une température comprise entre la température de 0°C et la température d'ébullition du solvant employé et l'on transforme éventuellement le produit ainsi obtenu dans ses sels pharmaceutiquement acceptables.

7. Procédé selon la revendication 6, caractérisé en ce que comme dérivé fonctionnel de l'acide on utilise un composé de formule

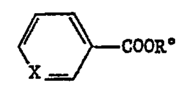

dans laquelle X a la signification donnée dans la revendication 6 et R° représente un groupe nitrophényle, méthoxyphényle, trityle, benzhydryle.

8. Procédé selon une des revendications 6 et 7, caractérisé en ce qu'on isole l'amide final sous forme d'un de ses sels et l'on libère la base libre dudit sel par neutralisation à l'aide d'un hydroxyde ou carbonate alcalin.

9. Composition pharmaceutique à action antagoniste des récepteurs $H_2$ de l'histamine renfermant, en tant qu'ingrédient actif, un composé selon l'une des revendications 1 à 5.

10. Composition pharmaceutique selon la revendication 9 sous forme d'unité de dosage, dans laquelle chaque unité de dosage contient de 10 à 1000 mg d'ingrédient actif en mélange avec un excipient pharmaceutique.

11. Composition pharmaceutique selon la revendication 10, contenant de 100 à 500 mg d'ingrédient actif par unité de dosage.

**Revendications** pour l'Etat contractant: AT

1. Procédé pour la préparation d'amides de formule

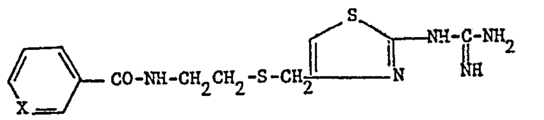

dans laquelle X représente un groupe ≥N→O ou un groupe ≥C−NH−A−B, où A représente un groupe CO ou $SO_2$ et B représente un groupe alkyle contenant de 1 à 6 atomes de carbone, un groupe phényle, un groupe pyridyle, un groupe pyridyle 1-oxyde, un groupe pyrazinyle ou un groupe thiényle, ainsi que de leurs sels pharmaceutiquement acceptables, caractérisé en ce que l'on traite la 2-(2-guanidinothiazol-4-ylméthylthio)éthylamine de formule

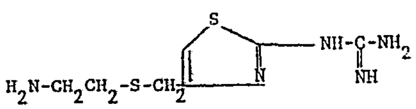

avec un dérivé fonctionnel d'un acide de formule

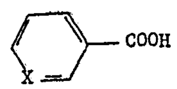

dans laquelle X est tel que défini ci-dessus, dans un solvant organique à une température comprise entre la température de 0°C et la température d'ébullition du solvant employé et l'on transforme éventuellement le produit ainsi obtenu en ses sels pharmaceutiquement acceptables.

2. Procédé selon la revendication 1, caractérisé en ce que comme dérivé fonctionnel de l'acide on utilise un composé de formule

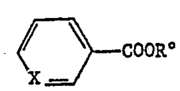

dans laquelle X a la signification donnée dans la revendication 1 et R° représente un groupe nitrophényle, méthoxyphényle, trityle, benzhydryle.

3. Procédé selon l'une des revendications 1 ou 2, caractérisé en ce qu'on isole l'amide final sous forme d'un de ses sels et l'on libère la base libre dudit sel par neutralisation à l'aide d'un hydroxyde ou carbonate alcalin.

4. Utilisation d'un amide obtenu par le procédé selon la revendication 1, pour la préparation d'une composition pharmaceutique à action antagoniste des récepteurs $H_2$ de l'histamine.

5. Utilisation selon la revendication 4, caractérisée en ce que l'amide est le N-[2-(2-guanidinothiazol-4-ylméthylthio)éthyl]-3-pyridinecarboxamide 1-oxyde ainsi que ses sels pharmaceutiquement acceptables.

6. Utilisation selon la revendication 4, caractérisée en ce que l'amide est le fumarate neutre de N-[2-(2-guanidinothiazol-4-ylméthylthio)éthyl]-3-pyridinecarboxamide 1-oxyde.

7. Utilisation selon la revendication 4, caractérisée en ce que l'amide est le 3-méthanesulfonamido-N-[2-(2-guanidinothiazol-4-ylméthylthio)-éthyl]benzamide et ses sels pharmaceutiquement acceptables.

8. Utilisation selon la revendication 4, caractérisée en ce que l'amide est le 3-benzènesulfonamido-N-[2-(2-guanidinothiazol-4-ylméthylthio)éthyl]benzamide et ses sels pharmaceutiquement acceptables.

**Patentansprüche** für die Vertragsstaaten BE, CH, DE, FR, GB, IT, LI, LU, NL, SE

1. Amide der Formel

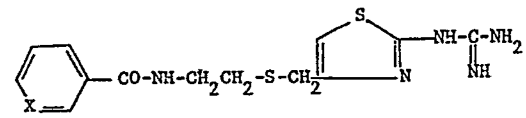

worin X eine Gruppe ≥N→O oder eine Gruppe ≥C−NH−A−B, in welcher A für eine CO- oder $SO_2$-Gruppe steht und B eine Alkylgruppe mit 1 bis 6 Kohlenstoffatomen, eine Phenylgruppe, eine Pyridylgruppe, eine Pyridyl-1-oxidgruppe, eine Pyrazinylgruppe oder eine Thienylgruppe bedeutet, darstellt, sowie deren pharmazeutisch akzeptable Salze.

2. N-[2-(2-Guanidinothiazol-4-yl-methylthio)-äthyl]-3-pyridincarboxamid-1-oxid sowie dessen pharmazeutisch akzeptable Salze.

3. Neutrales Fumarat von N-[2-(2-Guanidinothiazol-4-yl-methylthio)-äthyl]-3-pyridincarboxamid-1-oxid.

4. 3-Methansulfonamido-N-[2-(2-guanidinothiazol-4-ylmethylthio)-äthyl]benzamid und dessen pharmazeutisch akzeptable Salze.

5. 3-Benzolsulfonamido-N-[2-(2-guanidinothiazol-4-yl-methylthio)-äthyl]benzamid und dessen pharmazeutisch akzeptable Salze.

6. Verfahren zur Herstellung von Amiden der Formel

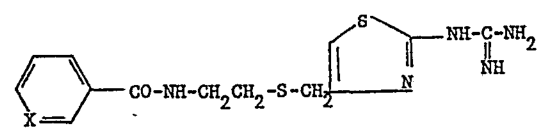

worin X eine Gruppe ≥N→O oder eine Gruppe ≥C−NH−A−B, in welcher A für eine CO- oder $SO_2$-Gruppe steht und B eine Alkylgruppe mit 1 bis 6 Kohlenstoffatomen, eine Phenylgruppe, eine Pyridylgruppe, eine Pyridyl-1-oxidgruppe, eine Pyrazinylgruppe oder eine Thienylgruppe bedeutet, darstellt, sowie von deren pharmazeutisch akzeptablen Salzen, dadurch gekennzeichnet, dass man 2-(2-Guanidinothiazol-4-yl-methylthio)äthylamin der Formel

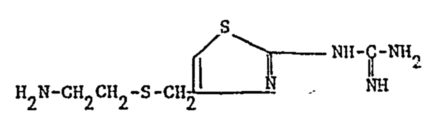

mit einem funktionellen Derivat einer Säure der Formel

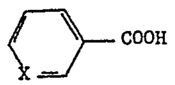

worin X obige Bedeutung hat, in einem organischen Lösungsmittel bei einer Temperatur zwischen 0° C und der Kochtemperatur des verwendeten Lösungsmittels behandelt und gegebenenfalls das so erhaltene Produkt in seine pharmazeutisch akzeptablen Salze überführt.

7. Verfahren nach Anspruch 6, dadurch gekennzeichnet, dass man als funktionelles Säurederivat eine Verbindung der Formel

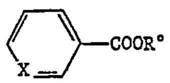

worin X die in Anspruch 6 angegebene Bedeutung hat und R° für eine Nitrophenyl-, Methoxyphenyl-, Trityl- oder Benzhydrylgruppe steht, verwendet.

8. Verfahren nach einem der Ansprüche 6 und 7, dadurch gekennzeichnet, dass man das End-Amin in Form eines seiner Salze isoliert und man die freie Base aus dem Salz durch Neutralisieren mittels eines Alkalihydroxids oder -carbonats freisetzt.

9. Pharmazeutische Zusammensetzung mit antagonistischer Wirkung auf die $H_2$-Rezeptoren von Histamin, welche als aktives Ingrediens eine Verbindung nach einem der Ansprüche 1 bis 5 enthält.

10. Pharmazeutische Zusammensetzung nach Anspruch 9 in Dosiseinheitsform, wobei jede Dosiseinheit 10 bis 1000 mg aktives Ingrediens in Mischung mit einem pharmazeutischen Exzipienten enthält.

11. Pharmazeutische Zusammensetzung nach Anspruch 10, welche 100 bis 500 mg aktives Ingrediens pro Dosiseinheit enthält.

**Patentansprüche** für den Vertragsstaat: AT

1. Verfahren zur Herstellung von Amiden der Formel

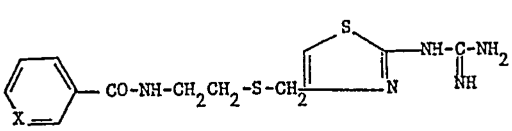

worin X eine Gruppe $\geqslant N \rightarrow O$ oder eine Gruppe $\geqslant C-NH-A-B$, in welcher A für eine CO- oder $SO_2$-Gruppe steht und B eine Alkylgruppe mit 1 bis 6 Kohlenstoffatomen, eine Phenylgruppe, eine Pyridylgruppe, eine Pyridyl-1-oxidgruppe, eine Pyrazinylgruppe oder eine Thienylgruppe bedeutet, darstellt, sowie von deren pharmazeutisch akzeptablen Salzen, dadurch gekennzeichnet, dass man 2-(2-Guanidinothiazol-4-yl-methylthio)äthylamin der Formel

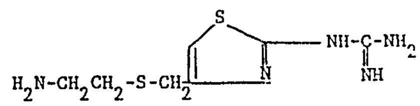

mit einem funktionellen Derivat einer Säure der Formel

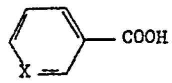

worin X obige Bedeutung hat, in einem organischen Lösungsmittel bei einer Temperatur zwischen 0° C und der Kochtemperatur des verwendeten Lösungsmittels behandelt und gegebenenfalls das so erhaltene Produkt in seine pharmazeutisch akzeptablen Salze überführt.

2. Verfahren nach Anspruch 1, dadurch gekennzeichnet, dass man als funktionelles Säurederivat eine Verbindung der Formel

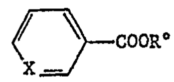

worin X die in Anspruch 1 angegebene Bedeutung hat und R° für eine Nitrophenyl-, Methoxyphenyl-, Trityl- oder Benzhydrylgruppe steht, verwendet.

3. Verfahren nach einem der Ansprüche 1 oder 2, dadurch gekennzeichnet, dass man das End-Amin in Form eines seiner Salze isoliert und man die freie Base aus dem Salz durch Neutralisieren mittels eines Alkalihydroxids oder -carbonats freisetzt.

4. Verwendung eines durch das Verfahren nach Anspruch 1 erhaltenen Amids zur Herstellung einer pharmazeutischen Zusammensetzung mit antagonistischer Wirkung auf die $H_2$-Rezeptoren von Histamin.

5. Verwendung nach Anspruch 4, dadurch gekennzeichnet, dass das Amid N-[2-(2-Guanidinothiazol-4-yl-methylthio)äthyl]-3-pyridincarboxamid-1-oxid sowie dessen pharmazeutisch akzeptable Salze ist.

6. Verwendung nach Anspruch 4, dadurch gekennzeichnet, dass das Amid das neutrale Fumarat von N-[2-(2-Guanidinothiazol-4-yl-methylthio)-äthyl]-3-pyridincarboxamid-1-oxid ist.

7. Verwendung nach Anspruch 4, dadurch gekennzeichnet, dass das Amid 3-Methansulfonamido-N-[2-(2-Guanidinothiazol-4-yl-methylthio)-äthyl]-benzamid und dessen pharmazeutisch akzeptable Salze ist.

8. Verwendung nach Anspruch 4, dadurch gekennzeichnet, dass das Amid 3-Benzolsulfonamido-N-[2-(2-guanidinothiazol-4-yl-methylthio)-äthyl]-benzamid und dessen pharmazeutisch akzeptable Salze ist.

**Claims** for the contracting States BE, CH, DE, FR, GB, IT, LI, LU, NL, SE

1. Amides of formula:

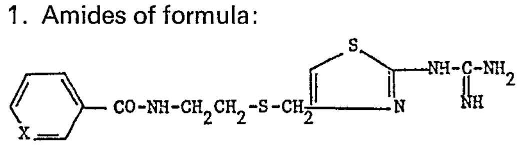

in which X represents a group $\geqslant N \rightarrow O$ or a group $\geqslant C-NH-A-B$ wherein A represents a CO or $SO_2$ group and B represents an alkyl group of from 1 to 6 carbon atoms or a phenyl, pyridyl group, pyridyl 1-oxide, pyrazinyl or thienyl group, and the pharmaceutically acceptable salts thereof.

2. The N-[2-(2-guanidinothiazol-4-ylmethylthio)ethyl]-3-pyridinecarboxamide 1-oxide and the pharmaceutically acceptable salts thereof.

3. The N-[2-(2-guanidinothiazol-4-ylmethylthio)ethyl]-3-pyridinecarboxamide 1-oxide neutral fumarate.

4. The 3-methanesulfonamido-N-[2-(2-guanidinothiazol-4-ylmethylthio)ethyl]benzamide and the pharmaceutically acceptable salts thereof.

5. The 3-benzenesulfonamido-N-[2-(2-guanidinothiazol-4-ylmethylthio)ethyl]benzamide and the pharmaceutically acceptable salts thereof.

6. A process for the preparation of amides of formula

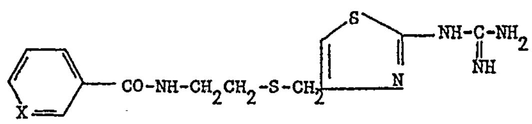

in which X represents a group $\geqslant N \rightarrow O$, or a group $\geqslant C-NH-A-B$, wherein A represents a CO or $SO_2$ group and B represents an alkyl group of from 1 to 6 carbon atoms or a phenyl, pyridyl, pyridyl 1-oxide, pyrazinyl or thienyl group, and of their pharmaceutically acceptable salts, characterized in that the 2-(2-guanidinothiazol-4-ylmethylthio)ethylamine of formula

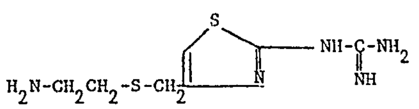

is treated with a functional derivative of an acid of formula

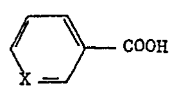

wherein X is as defined above, in an organic solvent at a temperature of from 0° C and the boiling temperature of the employed solvent and optionally converting the product thus obtained into their pharmaceutically acceptable salts.

7. A process according to claim 6, characterized in that a compound of formula

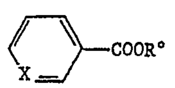

in which X is as defined in claim 6 and R° represents a nitrophenyl, methoxyphenyl, trityl or benzhydryl group, is used as functional derivative of the acid.

8. A process according to any one of claims 6 and 7, characterized in that the final amide is isolated in form of a salt thereof and the free base is splitted off from said salt by neutralization with an alkaline hydroxide or carbonate.

9. A pharmaceutical composition having histamine $H_2$ receptor antagonist action comprising, as active ingredient, a compound as claimed in one of the claims 1 to 5.

10. A pharmaceutical composition according to claim 9, which is in dosage unit form, comprising from 10 to 1000 mg of active ingredient in admixture with a pharmaceutical carrier.

11. A pharmaceutical composition according to claim 10, comprising from 100 to 500 mg of active ingredient per dosage unit.

**Claims** for the contracting State: AT

1. Process for the preparation of amides of formula

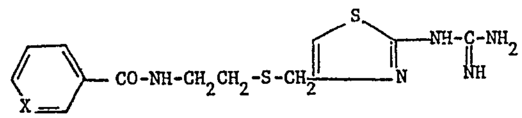

in which X represents a group $\geqslant N \rightarrow O$ or a group $\geqslant C-NH-A-B$, wherein A represents a CO or $SO_2$ group and B represents an alkyl group of from 1 to 6 carbon atoms or a phenyl, pyridyl group, pyridyl 1-oxide, pyrazinyl or thienyl group, and the pharmaceutically acceptable salts thereof, characterized in that the 2-(2-guanidinothiazol-4-ylmethylthio)ethylamine of formula

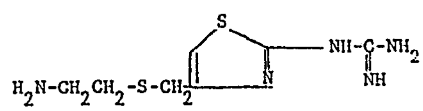

is treated with a functional derivative of an acid of formula

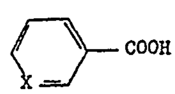

wherein X is as defined above, in an organic solvent at a temperature of from 0° C and the boiling temperature of the employed solvent and optionally converting the product thus obtained into their pharmaceutically acceptable salts.

2. A process according to claim 1, characterized in that a compound of formula

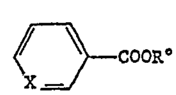

in which X is as defined in claim 1 and R° represents a nitrophenyl, methoxyphenyl, trityl or benzhydryl group, is used as functional derivative of the acid.

3. A process according to any one of claims 1 and 2, characterized in that the final amide is isolated in form of a salt thereof and the free base is splitted off from said salt by neutralization with an alkaline hydroxide or carbonate.

4. Use of an amide obtained by the process according to claim 1, for the preparation of a pharmaceutical composition having histamine $H_2$ receptor antagonist action.

5. Use according to claim 4, characterized in that the amide is the N-[2-(2-guanidinothiazol-4-ylmethylthio)ethyl]-3-pyridinecarboxamide 1-oxide and pharmaceutically acceptable salts thereof.

6. Use according to claim 4, characterized in that the amide is the N-[2-(2-guanidinothiazol-4-ylmethylthio)ethyl]-3-pyridinecarboxamide 1-oxide neutral fumarate.

7. Use according to claim 4, characterized in that the amide is the 3-methanesulfonamido-N-[2-(2-guanidinothiazol-4-ylmethylthio)ethyl]-benzamide and the pharmaceutically acceptable salts thereof.

8. Use according to claim 4, characterized in that the amide is the 3-benzenesulfonamido-N-[2-(2-guanidinothiazol-4-ylmethylthio)ethyl]-benzamide and the pharmaceutically acceptable salts thereof.